# EUROPEAN PATENT APPLICATION

(11) **EP 0 575 952 A1**
(43) Date of publication of application: **29.12.1993**
(21) Application number: 93109916.2
(22) Date of filing: 22.06.1993
(51) Int. Cl.: A61B 5/14

(54) **Blood sucking device**

(30) Priority: 26.06.1992 JP 50443/92 U
(71) Applicant: Suzuki, Yoshihiko, Tokyo-to (JP); NISSHO CORPORATION, Osaka-shi (JP); A & D CO., LTD., Toshima-ku, Tokyo-to (JP)
(72) Inventor: Suzuki, Yoshihiko, Tokyo-to (JP)
(74) Representative: Türk, Gille, Hrabal, Leifert

(57) **Abstract**

A blood sucking device is designed to be positioned on a skin portion (5) pierced with a lancet for collecting blood in a droplet form (5a) on the skin portion. The blood sucking device comprises a main tube (1) having an inner diameter larger than the diameter of a droplet of blood to be collected, a suction member (2) attached to the main tube, and an air-inlet aperture (3) provided at the main tube for introducing air into the main tube.

## Description

The present invention relates to a blood sucking device which is placed on a skin area having been pierced with a lancet so as to collect a droplet of blood for examination for various values such as of blood sugar, lactic acid, cholesterol, neutral fat, alcohol, oxygen, carbon dioxide, and pH.

A simple measuring device for determining a blood sugar value with ease is well known in which a chip electrode is inserted into an electrode-insertion opening of a main body of a hand-holdable small device, a tip electrode is impregnated with a droplet of blood which is collected on skin by means of a lancet, the blood is sucked into the main body utilizing capillary phenomenon, and result of determination is displayed on a display provided on the surface of the main body. It is proposed that such a determination at intervals of 10 to 15 minutes would contributes to development of an aritificial pancreas. In such blood collecting method, blood collection from a finger tip is most simple and permits to collect a necessary amount of blood with ease. However, the blood collection from the finger tip gives pain to the patient, and finger tips do not offer a sufficient skin area for repeated bood collections. For this reason, there is also a method wherein a necessary amount of blood is gotten by piercing an upper arm, trunk, thigh or the like and wringing the portion around the same. In order to collect a sufficient amount of blood with less pain in accordance with such a method, USP No. 4,653,513 has proposed a blood sampler which is designed to prick a skin by advancing a lancet supporter slidably supported in a tube and provided with a gasket therearound toward the skin and, in its returning stroke, to create a vacuum in the tube thereby sucking up blood.

With this blood sampler, however, when the tube is released from the skin against the suction force after collection of blood with the skin kept drawn up by vacuum, the blood is scattered around by a sudden restoring action of the skin and a sudden penetration of air into the tube. Accordingly, the blood sampler involves drawbacks that blood needs to be collected in an amount larger than necessary amount and the inside of the tube is smeared. Since velocity of the lancet in an advancing stroke is decreased due to friction of an airproofing member on the tube, such as a rubber seal, against the tube wall, the pain upon pricking the skin is increased. Further, the structure of the sampler is complicated, the manufacturing cost is relatively high, and the replacement of the lancet requires a relatively long time.

It is, therefore, an object of the present invention to provide a blood sucking device which is capable of quickly sucking out a sufficient amount of blood on the skin without collapsing a drop state of the blood after piercing with a lancet at a portion having a pain-point density and vascular density lower than a finger tip.

It is another object of the present invention to prevent scattering of the amount of blood collected under a given suction force by getting rid of a vacuum.

According to the present invention, there is provided a blood sucking device designed to be positioned on a skin portion pierced with a lancet for collecting blood in a droplet form on the skin portion,
the blood sucking device comprising a main tube having an inner diameter larger than the diameter of a droplet of blood to be collected, a suction member attached to the main tube, and an air-inlet aperture provided at the main tube for introducing air into the main tube.

According to the present invention, there is also provided a blood sucking device designed to be positioned on a skin portion pierced with a lancet for collecting blood in a droplet form on the skin portion,
the blood sucking device comprising a main tube having an inner diameter larger than the diameter of a droplet of blood to be collected, a suction member attached to the main tube, and a skin-wringing means provided at a tip portion of the main tube and designed to displace the skin portion elevated by suction of the suction member toward a center from a surroundings of the skin portion.

In one aspect of the present invention, immediately after piercing a portion other than the finger tip with a lancet, a peripheral tip portion of the main tube is brought into contact with the skin and inside of the main tube is sucked by the suction member with the air-inlet aperture closed, whereby blood is drawn out from the pierced portion and forms a droplet on the skin by lancet. The growing droplet of blood is observed through the transparent main tube, and the air-inlet aperture is opened to introduce air into the main tube when the droplet of blood has grown to a predetermined size. As a result, the suction of blood is stopped, the drawn skin resumes its usual position, and the peripheral tip portion of the main tube is released from the skin.

In another aspect of the present invention, blood is drawn out to form a droplet of blood on the skin, and if the amount of collected blood is found to be insufficient by the observation through the main tube, the amount of blood is increased by inwardly wringing the skin portion elevated by suction with the skin-wringing means; that is, by causing the skin-wringing means to displace so as to decrease the skin area surrounded thereby. After a droplet of blood in an desired amount is formed on the skin, vacuum for suction is broken to cause the elevated skin to resume its usual position and to release the peripheral tip portion of the main tube from adhesion to the skin. Alternatively, where the blood sucking device is always set for use and designed to operate automatically, the blood sucking device is previously positioned on a skin portion to be pierced with a lancet. Upon piercing by the lancet, the suction member is caused to automatically operate, and after a predetermined time, the skin-wringing means is actuated automatically, followed by automatically breaking the vacuum.
Fig. 1 is a front elevation showing a first embodiment of a blood sucking device according to the present invention;
Fig. 2 is a sectional view for illustrating the operation of the blood sucking device shown in Fig. 1;
Fig. 3 is a sectional view showing a valve of the blood sucking device shown in Fig. 1;
Fig. 4 is a front elevation showing a second embodiment of a blood sucking device according to the present invention;
Fig. 5 is a front elevation showing a third embodiment of a blood sucking device according to the present invention;
Fig. 6 is a sectional view showing a valve of the blood sucking device shown in Fig. 5;
Fig. 7 is a perspective view showing a fourth embodiment of a blood sucking device according to the present invention;
Fig. 8 is a front elevation showing a fifth embodiment of a blood sucking device according to the present invention;
Fig. 9 shows a partial structure of the blood sucking device shown in Fig. 8, wherein Fig. 9A is a plan view showing a main tube of the blood sucking device and Fig. 9B is a perspective view showing a portion to which a skin-wringing means is attached;
Fig. 10 is a perspective view showing a main portion of a sixth embodiment of a blood sucking device according to the present invention;
Fig. 11 is a sectional view showing a main portion of a seventh embodiment of a blood sucking device according to the present invention;
Fig. 12 is a plan view showing a bottom portion of the blood sucking device shown in Fig. 11;
Fig. 13 is a sectional view showing a main portion of an eighth embodiment of a blood sucking device according to the present invention; and
Fig. 14 is a sectional view through the tip of main tube taken along the line A-A of Fig. 13, wherein Fig. 14A is a state before a wringle operation and Fig. 14B is a state during a wringle operation.

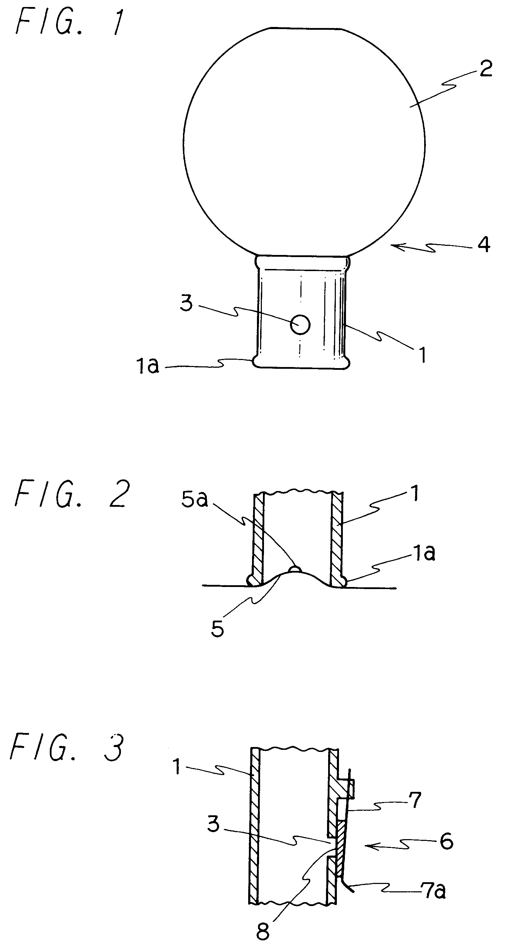

Referring to Fig. 1 showing a first embodiment of a blood sucking device according to the present invention, numeral 1 denotes a main tube made of synthetic resin whose inner diameter is made sufficiently larger than the diameter of a droplet of blood to be collected in a necessary amount, for example, about 20 mm. A flange 1a with a curved surface is formed at a peripheral tip portion of the main tube 1. An air-inlet aperture 3 having a diameter of, for example, about 2 mm is formed at a tube wall of the main tube 1. To the upper end of the main body 1 is fixed a rubber ball 2 as a suction member which is capable of restoring its spherical condition from its compressed condition by its own elasticity.

In using the blood sucking device 4 thus constructed, immediately after piercing, for example, a thigh portion with a lancet, the rubber ball 2 is compressed with fingers and the flange 1a is placed on the periphery of the pierced skin portion with the air-inlet aperture 3 closed with a finger so that the pierced portion would be located at substantially center of the circle defined by the main tube 1. The rubber ball 2 is then released from its compressed condition with the air-inlet aperture 3 still kept closed. This causes the rubber ball 2 to restore its spherical shape by its own elasticity. As a result, the flange 1a adheres to the skin by suction and a vacuum is created in the main tube 1. By the vacuum thus created, blood is sucked out from the pierced portion 5 and a droplet of blood 5a is formed gradually as shown in Fig. 2. While the state of the growing droplet of blood is observed through the main tube, the air-inlet aperture 3 is opened as soon as the droplet of blood has grown into a droplet 5a having a volume of, for example, about 2.5 to 5 µℓ, which is required for measurement by the aforementioned simple measuring device for determining a blood sugar value. This causes air to slowly flow into the main tube through the air-inlet aperture 3. The drawn-up skin restores its usual position gradually, and the flange 1a is released from the adhesion to the skin by suction. Thus, no force is produced to collapse the droplet 5a upon removal of the blood sucking device, and the scattering of the droplet 5a is avoided which would be caused upon forcedly removing the flange 1a from the drawn-up skin against the suction force.

As required, the air-inlet aperture 3 might be provided at the rubber ball 2. Further, a valve 6 as shown in Fig. 3 might be provided over the air-inlet aperture 3 for eliminating the need of closing it with a finger in the blood sucking operation. This valve comprises a plate spring 7 whose base end is fixed to the tube wall of the main tube 1, an elastic member 8 attached to the intermediate portion of the plate spring 7 for closing the air-inlet aperture 3, and an operating portion 7a formed at the other end of the plate spring 7 and bent so as to be operated with a finger. In the sucking operation by the rubber ball 2, the elastic member 8 is pressed against the air-inlet aperture 3 by the biasing force of the plate spring 7 to close it. Upon obtaining the droplet of blood 5a, the operation portion 7a is bent to go away from the aperture 3 whereby permitting air to flow into the tube.

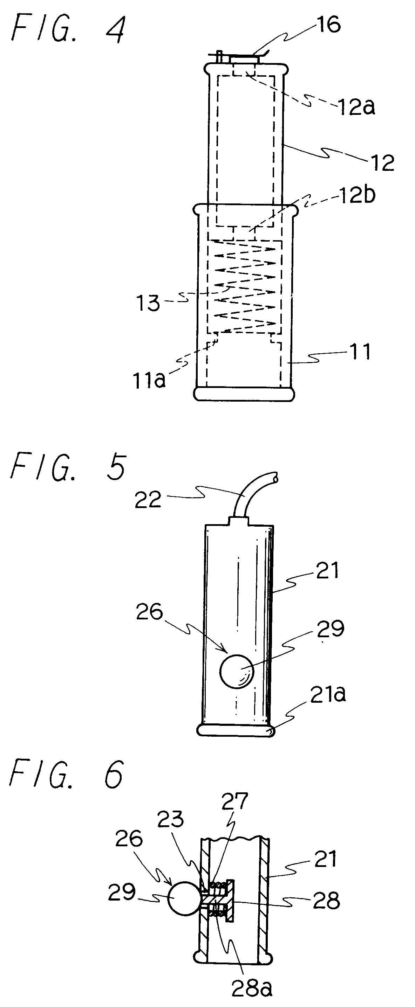

Fig. 4 illustrates a second embodiment of a blood sucking device according to the present invention. Referring to Fig. 4, in a main tube 11, there are provided a hollow planger 12 as the suction member which is slidable in the airtight condition, and a spring 13 for returning the planger 12 to its original position located above. The lower end of this spring 13 is engaged with a seat portion 11a formed in the tube, while the upper end thereof is engaged with the lower end of the planger 12. Air-inlet apertures 12a and 12b are formed at top and bottom walls of the planger 12 respectively for communication of open air therethrough. A valve 16 as shown in Fig. 3 is attached to the upper aperture 12a. Note that the aperture 12a might be provided with a valve of a different structure or designed to be closed with a finger without the provision of a valve.

Fig. 5 illustrates a third embodiment of a blood sucking device according to the present invention. Referring to Fig. 5, a main tube 21 having at its tip end a flange 21a is connected to an electric-operated vacuum pump (not shown) through a tube 22. The tube wall of the main tube 21 has an air-inlet aperture and is attached with a valve 26 which is shown in Fig. 6. The valve 26 comprises a compression spring 27 surrounding the aperture 23, a disk-like spring seat 28, and a spherical elastic member 29 attached to an end portion of a flat guide rod 28a which is projecting from the spring seat 28 and has arc side surfaces fitted to the perimeter of the aperture 23. The elastic member 29 closes the aperture 23 with the help of the spring 27 in the usual condition. The elastic member 29 is away from the tube wall and againt the spring 27 by drawing out with fingers to open the aperture 23.

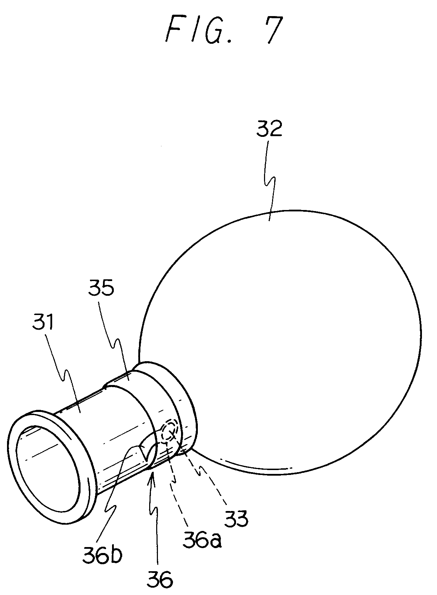

Fig. 7 illustrates a fourth embodiment of a blood sucking device according to the present invention. Referring to Fig. 7, a hollow rubber ball 32 is attached to an end of a main tube 31, and a rubber ring 35 is attached to an upper portion thereof. A valve comprises the rubber ring 35, and a lever 36 having a base portion 36a curved to fit the tube surface and close an air-inlet aperture 33, and an operating portion 36b on the side opposite to the base portion 36a. The base portion 36a is covered with the rubber ring 35 and, in the usual condition, closes the aperture 33. When the operating portion is raised away from the tube surface against the resilience of the rubber ring 35, air is introduced from the outside through the aperture 33.

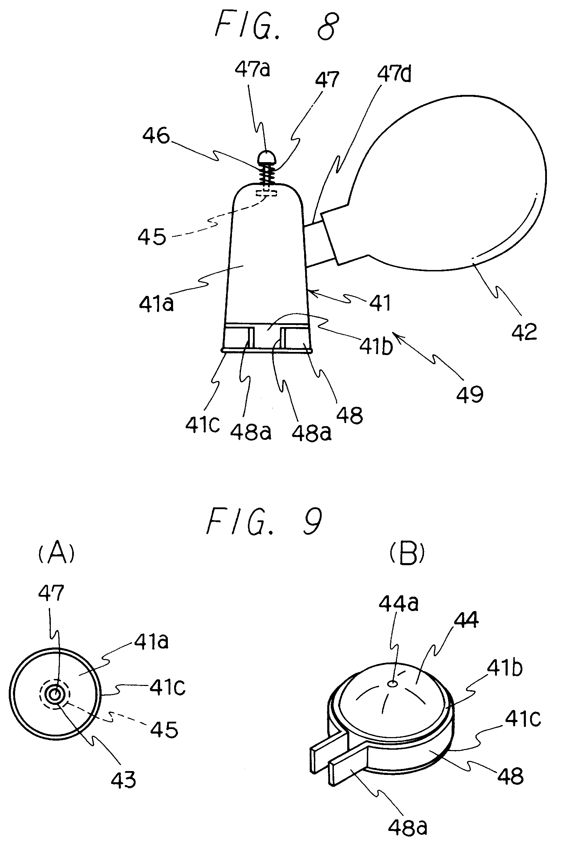

Figs. 8 and 9 illustrate a fifth embodiment of a blood sucking device according to the present invention. Referring to these figures, numeral 41 denotes a transparent main tube made of synthetic resin whose inner diameter is sufficiently larger than the diameter of a droplet of blood in a necessary amount, for example, about 20 mm. The main tube 41 comprises a rigid portion 41a, an elastic portion 41b forming a tip portion of the tube 41, and a rigid flange portion 41c of a ring-like shape provided on a peripheral tip of the tube 41. A branch tube 41d extends laterally and outwardly from the rigid portion 41a and is provided with a rubber ball 42 as the suction member, which can restore its original spheric condition from its compressed condition.

As shown in Fig. 9A, the top of the rigid portion 41a has an air-inlet aperture 43 through which a rod 47 is inserted. The rod 47 is provided at its front end with a valve 45 and at its base portion with a button 47a. The valve 45 is larger in diameter than the aperture 43. Between the aperture 43 and the button 47a is provided a spring 46 so as to cause the valve 45 to close the aperture 43 in the usual condition. A ring-shaped plate spring 48 as the skin-wringing means is mounted on the outer periphery of the elastic portion 41b. Both ends of the ring-shaped plate spring 48 are apart spaced at a distance corresponding to an open-close stroke and bent outwardly to form a knob 48a. These plate spring 48 and elastic portion 41b need not necessarily be transparent.

In using the blood sucking device 49 thus constructed, immediately after piercing, for example, a thigh portion with a lancet, the rubber ball 42 is compressed with fingers and the flange portion 41c is placed on the periphery of the pierced skin in the state that the aperture 43 is closed by the valve 45 so that the pierced portion would be located at substantially center of the circle defined by the main tube 41. The rubber ball 42 is then released from its compressed condition in the state that the aperture 43 is still kept closed. This causes the rubber ball 42 to restore its spherical shape by its own elasticity. As a result the flange portion 41c adheres to the skin, and a vacuum is created in the main tube 41. By the vacuum thus created, an elevated skin portion 44, which is elevated from the level of the surrounding skin, is formed. Blood is sucked out from the pierced portion at the center of the elevated skin portion 44 to form a droplet of blood 44 gradually, as shown in Fig. 9B.

In such a condition, pinching of the knob 48 causes the plate spring 48 to be further bent inwardly; that is, the inner diameter thereof is decreased. Thus, the elevated skin portion 44 is wrung from surroundings thereby easily collecting the droplet of blood 44a having a volume of, for example, about 2.5 to 5 µℓ which is necessary for measurement by the aforementioned measuring device for determining a blood sugar value. After the collection of blood, the button 47a is depressed against the biasing force of the spring 48 to open the aperture 43. Then, the aperture 43 permits air to flow into the tube 41 with a throttling function corresponding to the size of the aperture. As a result the elevated skin resumes its usual position, and the flange portion 41c is released from its adhesion to the skin. This produces no force to collapse the droplet of blood 44a upon removal of the blood sucking device 49 from the skin. Hence, the scattering of the droplet 44a is avoided which would be caused if the flange portion 41a adhering to the elevated skin is forcedly removed from the skin against the suction force.

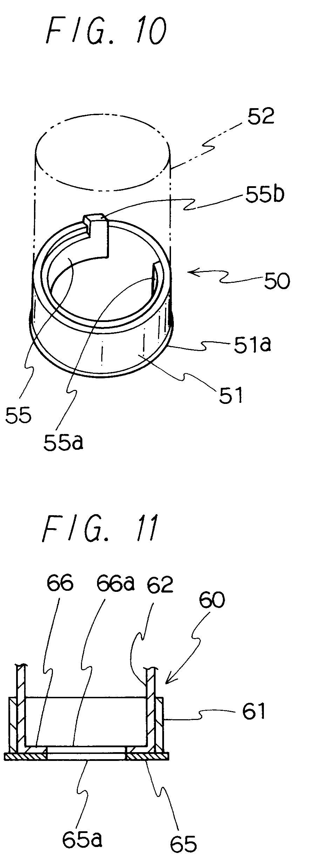

Fig. 10 illustrates a sixth embodiment of a blood sucking device according to the present invention. Referring to Fig. 10, a main tube 50 comprises a ring-shaped tube 51 provided at its tip portion with a flange portion 51a, and a tube portion 52 mounted airtightly and turnably on the outer periphery of the ring-like tube 51. The tube portion 52 is provided with a suction member and an air-inlet aperture. A skin-wringing means comprises an elastic ring 55 made of synthetic resin having spaced ends, one end 55a being fixed to the inner wall of the ring-shaped tube 51, the other end forming a reversed L-shaped mounting 55b which is fixed to the inner wall of the tube portion 52.

In using the blood sucking device thus constructed, the blood sucking operation is performed in the condition shown in the drawing. Subsequently the tube portion 52 is turned so that the both ends of the ring 55 would get closer to each other. This decreases the inner diameter of the ring 55 and wrings the elevated skin.

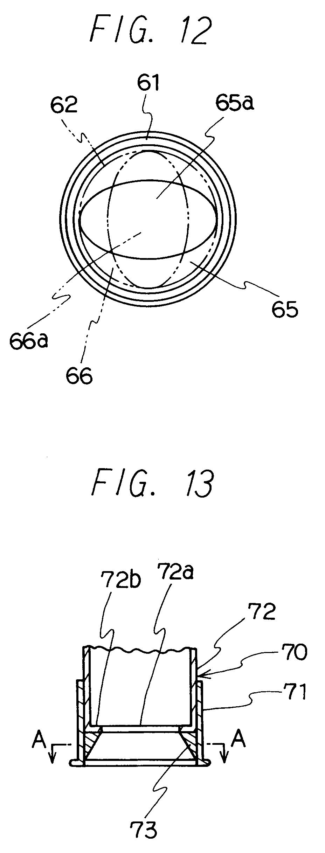

Figs. 11 and 12 illustrate a seventh embodiment of a blood sucking device according to the present invention. Referring to these figures, a main tube 60 comprises a ring-shaped tube 61 forming an outer peripheral tip portion of the main tube 60, and an inner tube portion 62 fitted airtightly and turnably into the ring-shaped tube 61. A skin-wringing means comprises a surface 65 forming a bottom of the ring-shaped tube 61 and having a substantially elliptic notch 65a, and a surface 66 forming a bottom of the tube portion 62 and having a substantially elliptic notch 66a which is identical with the notch 65a. The surface 66 is disposed on and in contact with the surface 65. Fig. 11 is a sectional view of this blood sucking device taken along the minor axis of the elliptic notches 65a and 66a.

When the tube portion 62 is turned with the skin sucked, the surface 66 is also turned, so that the opening defined by the notches 65a and 66a becomes small. Hence, the elevated skin is wrung gradually. The area of the opening is decreased to minimum when the tube portion 62 is turned by 90° (the condition shown in Fig. 12); thus, the skin is wrung most strongly at this position.

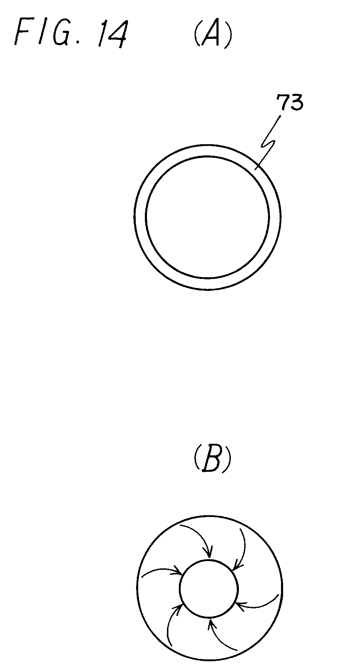

Figs. 13 and 14 illustrate an eighth embodiment of a blood sucking device according to the present invention. Referring to Fig. 13, a main tube 70 comprises a ring-shaped tube 71 forming an outer peripheral tip portion thereof, and an inner tube portion 72 fitted airtightly and turnably into the ring-shaped tube 71. A skin-wringing means comprises the main tube 70 and a tapered ring-shaped elastic member 73 whose top portion is stuck on an annular bottom surface 72b defining a center opening 72a of the tube portion 72, and whose bottom portion is stuck on an annular bottom edge defining a center opening of the tube portion. The inner diameter of the elastic member 73 increases gradually toward the bottom of the tube portion 71. With this construction, when the tube portion 72 is pressed down straightly or with being rotated, the elastic member 73 is compressed and the inner diameter of the opening which is taken along the line A-A of Fig. 13 is decreased gradually as shown in Fig. 14A and Fig. 14B, thereby providing a wringing action.

It should be understood that each of the tube portions 41a, 52 and 62 might be attached with any of various types of suction members including an automatic vacuum suction device and a hand-operated type suction device like an injector, as well as the rubber ball shown in Fig. 8. The respective air-inlet apertures of these tube portions need not necessarily be attached with a valve and might be closed with a finger. Further, the seventh and eighth embodiments of the blood sucking device might be constructed as an injector-type blood sucking device not having an air-inlet aperture, wherein the inner tube is drawn upward to create a vacuum, then returned to its original position to break the vacuum.

Where the blood sucking device of this invention is used in association with an artificial pancreas, the suction member should be an automatic suction device designed to automatically operate immediately after piercing the skin. Further, by providing the skin-wringing means to an actuator having a driving mechanism attached to a motor or the like, the skin-wringing means is designed to automatically operate in response to the operation of the suction member after a predetermined time. In that case the main tube need not necessarily be transparent.

As has been described, according to the present invention, after a skin portion other than finger tip is pierced with a lancet, the blood sucking device is positioned around the pierced portion to suck up blood. Hence, it is possible to stably collect a droplet of blood with less pain even from a portion having a low vascular concentration. Further, through a transparent main tube the state of collected blood can be monitored. Further, by opening the air-inlet aperture upon completion of collecting a necessary amount of blood while monitoring how much blood is collected, collection of blood in an amount required but not surplus can be assuredly achieved in accordance with individual patients from whom uniform-size droplets of blood are unexpected per unit time. Since the skin elevated by suction resumes its original position gradually to be released from the adhesion to skin by suction of air into the main tube, the scattering of blood can be avoided which would be caused if the tube is released from the skin being sucked against the suction force.

Further, according to the present invention, an increased amount of blood can be collected as compared with the case of the blood collection based only upon suction by wringing with the skin-wringing means after the completion of the sucking operation while being monitored through the transparent main tube, or by operating automatically the skin-wringing means after the completion of the sucking operation without monitoring the state of blood collection.

Though several embodiments of the present invention are described above, it is to be understood that the present invention is not limited only to the above-mentioned, various changes and modifications may be made in the invention without departing from the spirit and scope thereof.

## Claims

1. A blood sucking device designed to be positioned on a skin portion pierced with a lancet for collecting blood in a droplet form on the skin portion,
the blood sucking device comprising a main tube having an inner diameter larger than the diameter of a droplet of blood to be collected, a suction member attached to the main tube, and an air-inlet aperture provided at the main tube for introducing air into the main tube.

2. The blood sucking device of Claim 1, wherein a wall of the main tube comprises a transparent plastics.

3. The blood sucking device of any one of Claims 1 and 2, wherein a valve is provided at the air-inlet aperture for being manually operated to open the air-inlet aperture.

4. The blood sucking device of any one of Claims 1 to 3, wherein the suction member is a rubber ball mounted on an upper end of the main tube and communicating with the interior of the main tube.

5. The blood sucking device of any one of Claims 1 to 3, wherein the suction member comprises a planger inserted into the main tube, and a spring for returning the planger from its low position to its high position.

6. The blood sucking device of any one of Claims 1 to 3, wherein the suction member is a vacuum pump connected to the main tube through a tube.

7. The blood sucking device of any of Claims 1 to 6, wherein the device further includes a skin-wringing means which is provided at a tip portion of the main tube for displacing the skin portion elevated by suction of the suction member toward a center from a surroundings of the skin portion.

8. The blood sucking device of Claim 7, wherein the skin-wringing means is provided at the outer periphery of the flexible portion of the tip portion of the main tube and comprises an elastic ring whose both ends are spaced from each other and outwardly bent so as to be pinched by hand.

9. The blood sucking device of Claim 7, wherein the main tube comprises a ring-shaped first tube portion which forms a tip portion of the main tube, and a second tube portion which is fitted airtightly and turnably to the first tube portion; and wherein the skin-wringing means comprises a bendable and elastic ring having spaced ends, one of which being fixed to an inner wall of the first tube portion and the other of which being fixed to an inner wall of the second tube portion.

10. The blood sucking device of Claim 7, wherein the main tube comprises a ring-shaped first tube portion which forms a tip portion of the main tube, and a second tube portion which is fitted airtightly and turnably to an inner wall of the first tube portion; and wherein the skin-wringing means comprises a first surface forming a bottom of the first tube portion and having a substantially elliptic notch, and a second surface forming a bottom of the second tube portion and having a substantially elliptic notch which is identical in shape with the former, the second surface being disposed in contact with the downward first surface from upward.

11. The blood sucking device of Claim 7, wherein the main tube comprises a ring-shaped first tube portion which forms a tip portion of the main tube, and a second tube portion which is fitted airtightly and turnably to an inner wall of the first tube portion; and wherein the skin-wringing means comprises an elastic ring member which is attached to an annular bottom surface defining an opening of the second tube portion, an inner diameter of the ring member being gradually increased toward the tip thereof.
